# EUROPEAN PATENT APPLICATION

(11) **EP 0 626 451 A2**
(43) Date of publication of application: **30.11.1994**
(21) Application number: 94108113.5
(22) Date of filing: 26.05.1994
(51) Int. Cl.: C12N 15/85, C12N 15/16, C12N 5/10, C12P 21/00, C07K 15/06, C07K 15/08, A61K 37/24, C12Q 1/42

(54) **Heterodimers of a TGF-beta superfamily**

(30) Priority: 27.05.1993 JP 126368/93; 27.05.1993 JP 151388/93; 16.06.1993 JP 145241/93; 03.12.1993 JP 304248/93; 10.02.1994 JP 16796/94
(71) Applicant: Takeda Chemical Industries, Ltd., Osaka-shi, Osaka 541 (JP)
(72) Inventor: Fujisawa, Yukio, Higashinada-ku, Kobe, Hyogo 658 (JP); Hazama, Masatoshi, Ikeda, Osaka 563 (JP); Aono, Aki, Otsu, Shiga 520 (JP)
(74) Representative: von Kreisler, Alek, Dipl.-Chem.

(57) **Abstract**

A heterodimer composed of factors belonging to a TGF-β superfamily, and a composition for treatment of osteopathy containing the same, an expression vector which is autonomically replicable in an insect cell and contains one or more DNA(s) coding for a factor(s) belonging to a TGF-β superfamily, and a method for preparing the heterodimer having bone-inducing activity is disclosed.

## Description

### FIELD OF OF THE INVENTION

The present invention relates to a heterodimer composed of factors belonging to a TGF-β superfamily, and a composition for treatment of osteopathy containing the same. Further, the present invention relates to an expression vector which is autonomically replicable in an insect cell and contains one or more DNA(s) coding for a factor(s) belonging to a TGF-β superfamily, and a method for preparing the heterodimer having ectopic bone formation activity.

### BACKGROUND OF THE INVENTION

M. R. Urist reported that an intramuscular or a subcutaneous implant of decalcified bone matrixes induced ectopic bone formation [Science, 150, 893 (1965)], and a factor of this bone induction activity was named "bone morphogenetic protein (BMP)" due to its proteinaceous factor [M. R. Urist, Proc. Natl. Acad. Sci. U.S.A., 70, 3511 (1973)]. After the reports, many groups have attempted to purify the factor, but the substance had been unknown for a long time [A. H. Reddi et al., Proc. Natl. Acad. Sci. U.S.A., 69, 1601 (1972); A. H. Reddi. Collagen Rel. Res., 1, 209 (1981); T. Takaoka et al., Biomed. Res., 2, 466 (1981)]. In 1988, J. M. Wozney et al. purified the BNP based upon bone-inducing activity in vivo, and cloned four kinds of genes, human BMP-1, 2A (BMP-2), 2B (BMP-4) and BMP-3, using probes based on the partial amino acid sequence [Science, 242, 1528 (1989)]. Thereafter, their group further clarified the structure of BMP-5, 6, 7 and 8 genes [A. J. Celeste et al., Proc. Natl. Acad. Sci. U.S.A., 87, 9843 (1990); J. Cell Biochem. Suppl., 16F, 100 (1992)]. Osteogenin of F. P. Luyten et al. [J. Biol. Chem., 264, 13377 (1989)] is a molecule corresponding to BMP-3, osteogenic protein (OP)-1 of T. K. Sampath et al. [J. Biol. Chem., 265, 13198 (1990)] to BMP-7, Vgr-1 of K. Lyons et al. [Proc. Natl. Acad. Sci. U.S.A., 86, 4554 (1989)] to BMP-6, and mouse Dunn osteosarcoma-derived BMP of H. Yoshikawa and K. Takaoka [Jikken Igaku (Experimental Medicine), 10, 196 (1992)] to BMP-4, respectively.

In order to apply BMP to bone formation, recombinant BMP-2 has been prepared in a cultured animal cell.

Many factors belonging to the TGF-β superfamily are considered to function by processing precursors after expression of genes in vivo to finally form homodimers. Of these factors, inhibins α, β_{A} and β_{B} are known to form, by combination thereof, a heterodimer such as inhibin A (the heterodimer of α and β_{A}), inhibin B (the heterodimer of α and β_{B}) and actibin AB (the heterodimer of β_{A} and β_{B}), in addition to a homodimer such as actibin A (the homodimer of β_{A}) and actibin B (the homodimer of β_{B}). Each dimer shows different physiological action. Further, a heterodimer of TGF-β₁ and β₂ was isolated from the pig platelets and the decalcified bovine bones [S. Cheifetz et al., Cell, 48, 409 (1987); Y. Ogawa et al., J. Biol. Chem., 267, 2325 (1992), and the heterodimer of TGF-β₂ and β₃ was isolated from the decalcified bovine bones [Y. Ogawa et al., J. Biol. Chem., 267, 2325 (1992)].

WO93/09229 describes that a BMP heterodimer was produced in an animal cell by gene engineering techniques.

### SUMMARY OF THE INVENTION

The present invention provides a method for preparing a heterodimer composed of factors belonging to a TGF-β superfamily. Further, the present invention provides a heterodimer of factors belonging to a TGF-β superfamily and a composition capable of inducing osteogenesis.

For the purpose of creating a heterodimer stronger in specific activity than the conventional homodimers with an efficient method which produces little of each homodimer and can easily isolate a heterodimer from the mixture with homodimers, the present inventors have discovered that a heterodimer stronger in specific activity can be prepared by introducing an expression plasmid which expresses two different factors belonging to a TGF-β superfamily into a cultured insect cell.

The present invention provides
(1) an expression vector which is autonomically replicable in an insect cell and comprises one or more DNA(s) coding for a factor(s) belonging to a TGF-β superfamily;
(2) an expression vector which is autonomically replicable in an insect cell and comprises DNAs coding for two factors belonging to a TGF-β superfamily;
(3) the expression vector of (1) or (2) which is a Baculovirus vector;
(4) the expression vector of (1) or (2), wherein the insect cell is a Spodoptera litura-derived cell or a spanworm-derived cell;
(5) an insect cell transformed with the expression vector of (1);
(6) an insect cell transformed with the expression vector of (2);
(7) an insect cell transformed with an expression vector which is autonomically replicable in an insect cell and comprises a DNA coding for a factor belonging to a TGF-β superfamily and an expression vector which is autonomically replicable in an insect cell and comprises a DNA coding for a factor belonging to a TGF-β superfamily other than the above said factor;
(8) a method for preparing a heterodimer composed of two factors belonging to a TGF-β superfamily comprising the steps of cultivating the insect cell of (5), (6) or (7) in a medium under conditions sufficient for expression of the heterodimer, accumulating the heterodimer in a culture product, and collecting the resulting accumulated heterodimer.
(9) a heterodimer composed of two factors belonging to a TGF-β superfamily in which the first factor is drosophiladecapentapregic and the second factor is a factor belonging to a TGF-β superfamily other than the first factor;
(10) the heterodimer of (9), wherein the second factor is selected from the group consisting of bone morphogenetic protein 2, 3, 4, 5, 6, 7, 8 and 9, XenopusVg1 and drosophila 60A;
(11) a heterodimer composed of two factors belonging to a bone morphogenetic protein family which is derived from Xenopus laevis;
(12) a composition for osteogenesis which comprises a pharmaceutically acceptable carrier containing an effective amount of a heterodimer composed of two factors belonging to a TGF-β superfamily in which the first factor is drosophiladecapentapregic and the second factor is a factor belonging to a TGF-β superfamily other than the first factor;
(13) the composition of (12), wherein the second factor is selected from the group consisting of bone morphogenetic protein 2, 3, 4, 5, 6, 7, 8 and 9, XenopusVg1 and drosophila 60A;
(14) a composition capable of inducing osteogenesis which comprises a pharmaceutically acceptable carrier comprising an effective amount of a heterodimer composed of two factors belonging to a bone morphogenetic protein family which is derived from Xenopus laevis;
(15) a composition for therapy of osteopathy which comprises a pharmaceutically acceptable carrier comprising an effective amount of a heterodimer composed of two factors belonging to a TGF-β superfamily in which the first factor is drosophiladecapentapregic and the second factor is a factor belonging to a TGF-β superfamily other than the first factor;
(16) a composition for therapy of osteopathy which comprises a pharmaceutically acceptable carrier comprising an effective amount of a heterodimer composed of two factors belonging to a bone morphogenetic protein family which is derived from Xenopus laevis; and
(17) a method for screening and selecting compounds that affect alkaline phosphatase induction.

### BRIEF DESCRIPTION OF THE DRAWINGS

FIG. 1 is a schematic representation showing the construction of expression plasmid pEB4S obtained in Example 1;
FIG. 2 is a schematic representation showing the construction of expression plasmid pVLBM4-3 obtained in Example 2;
FIG. 3 is a schematic representation showing the construction of expression plasmid pVLBM7 obtained in Example 2; and
FIG. 4 is a schematic representation showing the construction of expression plasmid pVLBM2/4 obtained in Example 2.

### DESCRIPTION OF THE PREFERRED EMBODIMENTS

In the present invention, a TGF-β superfamily includes a bone morphogenetic protein (hereinafter also briefly referred to as "BMP") family, a transforming growth factor (hereinafter also briefly referred to as "TGF-β") family and an inhibin family.

The factors belonging to the BMP family include BMP-2, -3, -4, -5, -6, -7, -8 and -9, XenopusVg1, drosophiladecapentapregic (hereinafter also briefly referred to as "DPP") and drosophila 60A. Of these, BMP-4, -6 and -7, and drosophiladecapentapregic are preferred. The factors belonging to the inhibin family include inhibins α, β_{A} and β_{B}. The factors belonging to the TGF-β family include TGF-β₁, β₂, β₃, β₄ and β₅. TGF-β₁ and β₂ are preferred.

The factors belonging to the TGF-β superfamily include factors derived from mammals (for example, humans, cattle, pigs, chickens, mice and rats), amphibians (for example, Xenopus) and arthropods (for example, drosophilas).

The factors belonging to the TGF-β superfamily of the present invention may be of the natural type or muteins. There in no particular limitation on the muteins, as long as they have bone morphogenetic action, the amino acid sequences of original proteins are mutated by addition of amino acids, deletion of constituent amino acids or substitution of constituent amino acids with other amino acids to obtain the muteins.

Such addition includes addition of at least one amino acid. Such deletion includes deletion of at least one constituent amino acid. Such substitution includes substitution of at least one constituent amino acid with another amino acid.

At least one amino acid in the addition type mutein excludes methionine which is an initiation codon used for peptide expression, or a signal peptide. The number of amino acids added is at least one, but it may be any, as long as the activity is not lost.

The number of constituent amino acids deleted in the mutein which lacks at least one constituent amino acid may be any, as long as the characteristics of the natural type factor are not lost.

The number of constituent amino acids before substitution in the mutein which has another amino acid substituted for at least one constituent amino acid may be any, as long as the characteristics of the natural type factor are not lost.

The mutein may further be mutated by combinations of two or three of the above-mentioned addition, deletion and substitution.

cDNA nucleotide sequences of the factors belonging to the TGF-β superfamily used in the present invention can be prepared by amplification using polymerase chain reaction (hereinafter also sometimes briefly referred to as "PCR") based on the known sequences. For example, the cDNA nucleotide sequences of xBMP-2, 4 and 7 derived from Xenopus (hereinafter also sometimes briefly referred to as "x") are reported in Japanese Patent Unexamined Publication No. 4-154799 and S. Nishimatsu et al., Biochem. Biophys. Res. Commun., 186, 1487 (1992). The cDNA nucleotide sequences of BMP-2, 4, 6 and 7 derived from humans are reported by J. M. Wozney et al., Science, 242, 1528 (1988) and A. J. Celeste et al., Proc. Natl. Acad. Sci. U.S.A., 87, 9843 (1990). The cDNA nucleotide sequence of DPP derived from drosophila is reported by R.W. Padgett et al., Nature 325, 81(1987).

The cDNA nucleotide sequence of human BMP-9 is disclosed in WO 9300432 (1993).

The cDNA nucleotide sequence of drosophila 60A is reported by K.A. Wharton et al., Proc. Natl. Acad. Sci. U.S.A., 88, 9214 (1991).

The cDNA nucleotide sequence of XenopusVg1 is reported by D.L. Weeks and D.A. Melton, Cell, 51, 861 (1987).

The cDNA nucleotide sequences of α, β_{A} and β_{B} subunits of human-derived inhibin are reported by A. J. Mason et al., Biochm. Biophys. Res. Commun., 135, 957 (1986).

The cDNA nucleotide sequence of activin β_{B} of Xenopus laevis is reported by C.E. Dohrmann et al., Dev. Biol., 157, 474 (1993).

The cDNA nucleotide sequence of human-derived TGF-β is reported by R. Derynck et al., Nature, 316, 701 (1985). The cDNA nucleotide sequence of TGF-β2 of Xenopus laevis is reported by P. Kondaiah et al., Nucleic Acids Res., 18, 2185 (1990). The cDNA nucleotide sequence of TGF-β5 is reported by P. Kondaiah et al., J. Biol. Chem.,, 265, 1089 (1990).

In the present invention, heterodimers of the TGF-β superfamily can be prepared by genetic engineering techniques using an insect cell, preferably derived from a Spodoptera litura or a spanworm as a host cell.

The heterodimer of the present invention can be prepared by cultivating in a medium the insect cell transformant bearing the vector which is autonomically replicable in an insect cell and contains the DNA coding for factors belonging to the TGF-β superfamily, accumulating the heterodimer in a culture medium, and collecting the resulting accumulated heterodimer.

As to the vector containing the DNA coding for two factors belonging to the TGF-β superfamily, the two factors may be incorporated into one kind of vector concurrently, or into two kinds of vectors, respectively.

For example, the heterodimer of the TGF-β superfamily of the present invention or the mutein thereof can be obtained by concurrently expressing the cDNAs coding for the two different factors belonging to the TGF-β superfamily in cultured insect cells. This method comprises, for example, the steps of (1) synthesizing a primer for PCR to amplify the nucleotide sequence of the TGF-β superfamily cDNA previously reported, (2) preparing a recombinant expression plasmid for expressing the amplified TGF-β superfamily cDNAs by the use of the cultured insect cells, (3) preparing a cultured insect cell transformed with the recombinant expression plasmid described in (2), and (4) cultivating the insect cell in a medium and separating and purifying the heterodimer from a culture medium.

The above-described method for preparing a heterodimer which uses an insect cell as a host, produces little homodimers derived from each factor and can efficiently obtain the heterodimer.

PCR can be conducted by adding a sense primer and an anti-sense primer synthesized based on the cDNA nucleotide sequences previously reported, according to known methods, for example, according to the instruction of a Cetus/Perkin-Elmer kit. The amplified cDNA can be recovered from a gel after sepatation by known methods, for example, by agarose electrophoresis. The nucleotide sequence of this cDNA can be determined by the dideoxynucleotide synthetic chain termination method [T. Messing et al., Nucl. Acids Res., 9, 309 (1981)]. The plasmid having the cloned cDNA can be used for insertion into another vector as it is, or optionally after cutting out with an appropriate restriction enzyme.

Any vector may be used as long as it can be replicated in an insect cell. Examples of such vectors include Baculovirus transfer vectors pVL1392, pVL1393 and pBlueBac [the manual (MAXBAC™ Baculovirus expression system, Manual version 1.4) of the manufacturer (Invitrogen Corporation, CA, U.S.A.].

The cloned cDNA may have a translation initiation codon (ATG) at the 5'-terminus thereof, and a translation termination codon (TAG, TGA or TAA) at the 3'-terminus thereof. A promoter sequence is further ligated upstream therefrom to express the cDNA.

The promoter used in the present invention may be any as long as it is suitable for expression in an insect cell. Examples of such promoters include a polyhedrin promoter of a nuclear polyhedrosis virus.

The promoters can be prepared from the corresponding genes, and can also be chemically synthesized.

As a signal sequence and a pre-pro sequence, it is preferred to use a sequence inherent to the gene of each factor belonging to the TGF-β superfamily. However, any sequence may be used as long as it functions in a host.

By using the DNA-containing recombinant expression plasmid thus constructed, the transformant is prepared.

The hosts may be any insect cell. The insect cells include cells derived from Spodoptera litura (such as Sf9 (Spodoptera frugiperda 9) cell and Sf21 cell) and cells derived from spanworms (such as 5B1-4 (High Five) cell) which are all available from Invitrogen Corporation.

The transformation of the insect cells is conducted according to the manual (MAXBAC™ Baculovirus expression system, Manual version 1.4) of the manufacturer (Invitrogen Corporation)]. The transformants thus obtained are cultivated by per se known methods.

When the insect cell transformants are cultivated, media used for cultivation include, for example, TNM-FH medium [W. F. Hink et al., Nature, 226, 466 (1990)]. The cultivation is usually carried out at about 15°C to about 30°C for about 24 hours to about 72 hours, with aeration or agitation if necessary.

In the present invention, the expressed products can be isolated from the above-mentioned culture medium by appropriate combinations of per se known separating and purifying methods. These known separating and purifying methods include methods utilizing solubility such as salt precipitation and solvent precipitation, methods mainly utilizing a difference in molecular weight such as dialysis, ultrafiltration, gel filtration and SDS-polyacrylamide gel electrophoresis (SDS-PAGE), methods utilizing a difference in electric charge such as ion-exchange chromatography, methods utilizing specific affinity such as affinity chromatography, methods utilizing a difference in hydrophobicity such as reverse phase high performance liquid chromatography, and methods utilizing a difference in isoelectric point such as isoelectro-focussing electrophoresis.

According to the present invention, the heterodimer composed of two factors belonging to the TGF-β superfamily can be prepared with high purity and in large amounts.

Of the heterodimers of the present invention, a heterodimer composed of two factors belonging to a TGF-β superfamily in which the first factor is drosophiladecapentapregic and the second factor is a factor belonging to a TGF-β superfamily other than the first factor is a novel substance. The second factor is preferably selected from the group consisting of bone morphogenetic protein 2, 3, 4, 5, 6, 7, 8 and 9, XenopusVg1 and drosophila 60A, more preferably is bone morphogenetic protein 4, 6 or 7, especially 7.

The heterodimer composed of two factors belonging to a bone morphogenetic protein family which is derived from Xenopus laevis is also a novel substance. In the heterodimer, the bone morphogenetic protein family includes bone morphogenetic protein 2, 3, 4, 5, 6, 7, 8 and 9, XenopusVg1, drosophiladecapentapregic and drosophila 60A. Among them, bone morphogenetic protein 4, 6 or 7 is preferable, of which examples include the combination of bone morphogenetic proteins 4 and 6, or 4 and 7.

While the above described novel heterodimers are preferably produced according to the methods of the present invention, the following methods also can be used for producing them.

The heterodimer of the present invention can be prepared by cultivating, in a medium, the transformant bearing the vector which contains the DNAs coding for two factors, accumulating the heterodimer in a culture medium, and collecting the resulting accumulated heterodimer.

As to the vector containing the DNA coding for two factors belonging to the TGF-β superfamily, the two factors may be incorporated into one kind of vector concurrently, or into two kinds of vectors, respectively.

For example, the novel heterodimer of the present invention or the mutein thereof can be obtained by concurrently expressing the cDNAs coding for the two different factors constituting the heterodimer in cultured vertebrate cells, which comprises, for example, the steps of (1) synthesizing a primer for PCR to amplify the nucleotide sequence of the cDNA previously reported, (2) preparing a recombinant expression plasmid for expressing the amplified cDNAs by the use of the cultured vertebrate cells, (3) preparing a cultured vertebrate cell transformed with the recombinant expression plasmid described in (2), and (4) cultivating the vertebrate cell in a medium and separating and purifying the heterodimer from a culture medium.

PCR can be conducted by adding a sense primer and an anti-sense primer synthesized based on the cDNA nucleotide sequences previously reported, according to known methods, for example, according to the instruction of a Cetus/Perkin-Elmer kit. The amplified cDNA can be recovered from a gel after sepatation by known methods, for example, by agarose electrophoresis. The nucleotide sequence of this cDNA can be determined by the dideoxynucleotide synthetic chain termination method [T. Messing et al., Nucl. Acids Res., 9, 309 (1981)]. The plasmid having the cloned cDNA can be used for insertion into another vector as it is, or optionally after cutting out with an appropriate restriction enzyme.

Any vector may be used as long as it can be replicated in a host. When the host is Escherichia coli, examples of such vectors include plasmids derived from E. coli such as pBR322 [F. Bolivar et al., Gene, 2, 95 (1979)], pBR325 pUC12 and pUC13. When the host is yeast, examples of such vectors include pSH19 [S. Harashima et al., Mol. Cell. Biol., 4, 771 (1984)] and pSH19-1 (European Patent Publication No. EP-A-0235430). When the host is a vertebrate cell, examples of such vectors include pSV2-X [R. C. Mulligan and P. Berg, Proc. Natl. Acad. Sci. U.S.A., 78, 2072 (1981)] in which ori of SV40 is inserted in pBR322, and pcD-X [H. Okayama and P. Berg, Mol. Cell. Biol., 3, 280 (1983)].

The cloned cDNA may have a translation initiation codon (ATG) at the 5'-terminus thereof, and a translation termination codon (TAG, TGA or TAA) at the 3'-terminus thereof. A promoter sequence is further ligated upstream therefrom to express the cDNA.

The promoter used in the present invention may be any as long as it is suitable for expression in a host selected for the gene expression. When the host is E. coli, examples of such promoters include a T7 promoter, a trp promoter, a tac promoter, a lac promoter and an λPL promoter. The T7 promoter is preferred among others. When the host is yeast, examples of such promoters include a GAPDH promoter, a PGK promoter, a PHO5 promoter and an ADH promoter. The GAPDH promoter is preferred among others. When the host is a vertebrate cell, examples of such promoters include a SV40-derived promoter, a retrovirus promoter and a human cytomegalovirus promoter.

The promoters can be prepared from the corresponding genes, and can also be chemically synthesized.

As a signal sequence and a pre-pro sequence, it is preferred to use a sequence inherent to the gene of each factor belonging to the TGF-β superfamily. However, any sequence may be used as long as it functions in a host.

By using the DNA-containing recombinant expression plasmid thus constructed, the transformant is prepared.

The hosts include, for example, Escherichia, yeast and vertebrate cells. Examples of Escherichia include E. coli K12 DH1 [B. Low, Proc. Natl. Acad. Sci. U.S.A., 60, 160 (1968)], C600 [R. K. Appleyard, Genetics, 39, 440 (1954)], MM294 [K. Backman et al., Proc. Natl. Acad. Sci. U.S.A., 73, 4174 (1976)] and N4830 [J. Mol. Biol., 140, 57, (1980)]. Examples of the yeast include Saccharomyces cerevisiae AH22R⁻ [A. Miyanohara et al., Proc. Natl. Acad. Sci. U.S.A., 80, 1 (1983)], NA87-11A, DKD-5D, NA74-3A, NA74-3Aρ⁻ [Y. Kaisho et al., Yeast, 5, 91 (1989)], Schizosaccharomyces pombe ATCC38399 (h⁻ leul-32) and TH168 (h⁹⁰ ade6-M210 ural leul) [M. Kishida and C. Shimada, Current Genetics, 10, 443 (1986)]. Examples of the vertebrate cells include adherent cells such as monkey cell COS-7, monkey cell vero, Chinese hamster ovary (CHO) cell, mouse L cell and human FL cell, and non-adherent cells such as mouse myeloma cell (Sp2/O cell, etc.), mouse YAC-1 cell, mouse MethA cell, mouse P388 cell and mouse EL-4 cell.

The transformation of Escherichia described above is carried out, for example, according to the method described in T. Maniatis et al., Molecular Cloning, p.249, Cold Spring Harbor Laboratory (1982). The transformation of the yeast is conducted, for example, according to the method described in A. Hinnen et al., Proc. Natl. Acad. Sci. U.S.A., 75, 1929 (1978)]. The transformation of the vertebrate cells is carried out, for example, according to the method described in M. Wigler et al., Cell, 14, 725 (1978).

The transformants thus obtained are cultivated by per se known methods.

When the Escherichia transformants are cultivated, media used for cultivation include, for example, M9 medium containing glucose and Casamino Acids [J. H. Miller et al., Experiments in Molecular Genetics, page 431, Cold Spring Harbor Laboratory (1972)] is preferably used. In order to stimulate the promoter activity, a drug such as isopropyl thiogalactoside (IPTG) or indolyl-3-acrylic acid may be added thereto if necessary. The cultivation is usually carried out at about 15^{o} to about 43°C for about 3 to about 24 hours, with aeration or agitation if necessary.

When the yeast transformants are cultivated, examples of media used for cultivation include Burkholder minimum medium [K. L. Bostian et al., Proc. Natl. Acad. Sci. U.S.A., 77, 4504 (1980)]. The pH of the media is preferably adjusted to about 5 to about 8. The cultivation is usually carried out at about 20^{o} to about 35°C for about 24 to about 72 hours, with aeration or agitation if necessary.

When the vertebrate cell transformants are cultivated, media used for cultivation include, for example, MEM medium supplemented with about 5 to about 20% fetal calf serum [H. Eagle, Science, 130, 432 (1959)], DMEM medium [R. Dulbecco and G. Freeman, Virology, 8, 396 (1959)], RPMI-640 medium [G. E. More et al., J. Am. Med. Assoc., 199, 519 (1967)], 199 medium [J. F. Morgan, et al., Proc. Soc. Biol. Med., 73, 1 (1950)] and ASF104 medium (Ajinomoto Co., Inc.). The cultivation is usually carried out at about 30° to about 40°C for about 15 to about 60 hours, with aeration or agitation if necessary.

In the present invention, the expressed products can be isolated from the above-mentioned culture medium by appropriate combinations of per se known separating and purifying methods. These known separating and purifying methods include methods utilizing solubility such as salt precipitation and solvent precipitation, methods mainly utilizing a difference in molecular weight such as dialysis, ultrafiltration, gel filtration and SDS-polyacrylamide gel electrophoresis (SDS-PAGE), methods utilizing a difference in electric charge such as ion-exchange chromatography, methods utilizing specific affinity such as affinity chromatography, methods utilizing a difference in hydrophobicity such as reverse phase high performance liquid chromatography, and methods utilizing a difference in isoelectric point such as isoelectro-focussing electrophoresis.

According to the above-described methods, the novel heterodimer of the present invention can be produced.

The heterodimer of the present invention induces the production of alkaline phosphatase activity which is an indication of calcification of osteoblasts.

The heterodimers of the present invention are utilized as medicines for mammals (such as humans, mice, rats, cats, dogs, rabbits, cattle and pigs), because of their high bone-inducing activity, and can be used, for example, as bone-inducing agents when the bones are repaired or transplanted. The heterodimer of the present invention can also be used for a treatment of non-connective fracture, a fixation of artificial arthrosis and a repair of an alveolar bone. For example, the heterodimers of the present invention can be allowed to be adhered to or contained in artificial bones made of metals, ceramics or polymers. The artificial bones preferably have porous surfaces so that the heterodimers are released in the organism tissues in transplanting the artificial bones to bone defective portions.

The heterodimers of the present invention are dispersed in appropriate dispersing agents, binders and diluents, etc. such as collagen, physiological saline, citric acid solutions, acetic acid solutions, hydroxyapatite, fibrin and mixed solutions thereof. The artificial bones can be coated or impregnated with the resulting dispersions, and then dried, thereby allowing the heterodimers to be adhered to or contained in the artificial bones. Such artificial bones are transplanted to bone-defective portions, and firmly fixed thereto. Fixing agents for artificial bones can be prepared by mixing the heterodimers, active ingredients, with physiologically acceptable dispersing media, binders, diluents, other ingredients effective for osteoanagenesis (for example, calcium), etc. The fixing agents for artificial bones can also be used so as to fill gaps between the artificial bones transplanted to bone-defective portions and the bone-defective portions, without allowing the fixing agents to be adhered to or contained in the artificial bones.

The heterodimers of the present invention are low in toxicity and can be safely used. For example, they can be topically given in an amount of 0.1 to 100 mg, preferably 0.1 to 10 mg, to bone-defective portions or -decreased portions.

The heterodimers of the present invention can also be used in a simple in vitro assay for screening and selecting compounds that affect alkaline phosphatase induction. The screening method involves adding a heterodimer of the present invention to an in vitro assay suitable for measuring alkaline phosphatase induction in a cell e.g., an osteoblast, such as the method set forth in Experimental Example 1. The amount of alkaline phosphatase induction by the heterodimers recorded. The test compound is then added to the assay and alkaline phosphatase induction is again measured. Test for compounds can include, for example, proteins, chemicals or drugs. Any change in alkaline phosphatase induction activity of the heterodimer would indicate that the test compound has an influence on alkaline phosphatase induction. Such compounds may then be further evaluated.

When nucleotides, amino acids and so on are indicated by abbreviations in the specification and drawings, the abbreviations adopted by the IUPAC-IUB Commission on Biochemical Nomenclature or commonly used in the art are employed. For example, the following abbreviations are used. When the amino acids are capable of existing as optical isomers, it is understood that the L-forms are represented unless otherwise specified.
- DNA: : Deoxyribonucleic acid
- A: : Adenine
- T: : Thymine
- G: : Guanine
- C: : Cytosine
- SDS: : Sodium dodecyl sulfate
- Gly: : Glycine (G)
- Ala: : Alanine (A)
- Val: : Valine (V)
- Leu: : Leucine (L)
- Ile: : Isoleucine (I)
- Ser: : Serine (S)
- Thr: : Threonine (T)
- Cys: : Cysteine (C)
- 1/2 Cys: : Half cysteine
- Met: : Methionine (M)
- Glu: : Glutamic acid (E)
- Asp: : Aspartic acid (D)
- Lys: : Lysine (K)
- Arg: : Arginine (R)
- His: : Histidine (H)
- Phe: : Phenylalanine (F)
- Tyr: : Tyrosine (Y)
- Trp: : Tryptophan (W)
- Pro: : Proline (P)
- Asn: : Asparagine (N)
- Gln: : Glutamine (Q)
- Apr: : Ampicillin-resistant gene
- Tcr: : Tetracycline-resistant gene
The present invention will be described in more detail with the following Examples and Experimental Examples. It is understood of course that they are for the purpose of illustration only and are not intended to limit the scope of the invention.

E. coli HB101 carrying Baculovirus transfer plasmid pVLBM4-3, E. coli HB101 carrying Baculovirus transfer plasmid pVLBM7, and E. coli HB101 carrying Baculovirus transfer plasmid pVLBM2/4 prepared in Example 2 described below were deposited with the Institute for Fermentation, Osaka, Japan (IFO) and with the National Institute of Bioscience and Human-technology (formerly the Fermentation Research Institute), the Agency of Industrial Science and Technology, the Ministry of International Trade and Industry, Japan (NIBH). The accession numbers and the deposit dates are shown in Table 1.

**TABLE 1**

| Transformant | IFO | NIBH |
|---|---|---|
| E. coli HB101/ pVLBM4-3 | IFO 15486 (May 25, 1993) | FERM BP-4312 (May 31, 1993) |
| E. coli HB101/ pVLBM7 | IFO 15487 (May 25, 1993) | FERM BP-4314 (May 31, 1993) |
| E. coli HB101/ pVLBM2/4 | IFO 15488 (May 25, 1993) | FERM BP-4313 (May 31, 1993) |

### Example 1

### Preparation of Rabbit Anti-xBMP4 Antibody

Restriction enzymes, modifying enzymes and linkers used for recombinant DNAs were all purchased from New England Biolabs (Beverly, MA, U.S.A.)

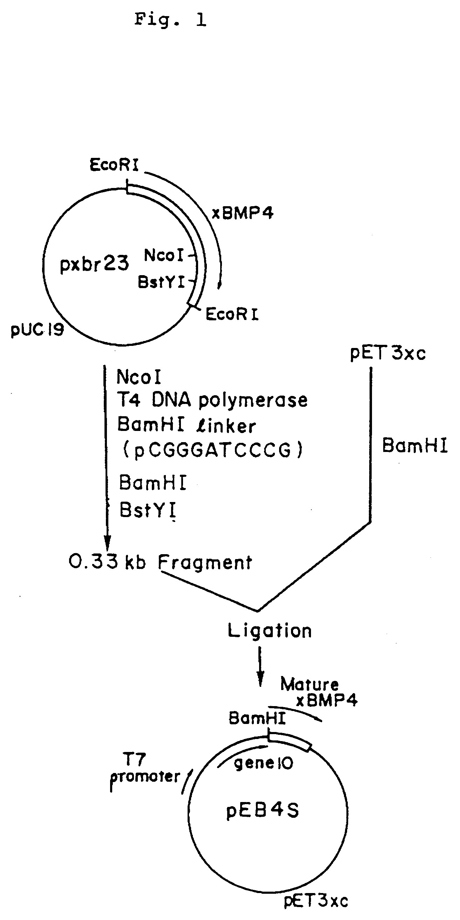

cDNA of xBMP4 (pXbr23, Japanese Patent Unexamined Publication No. 4-154799) was cleaved with restriction enzyme NcoI, and an end was rendered flush with a Klenow fragment. Then, a BamHI linker [pCGGGATCCCG; SEQ ID NO: 1] was added thereto, followed by treatment with restriction enzyme BstYI, thus separating a DNA fragment coding for a part (³³¹His-⁴⁰⁰Arg) of mature xBMP4. The resulting fragment was inserted into the BamHI site of vector pET3xc to prepare an expression plasmid pEB4S (Fig. 1). A transformant obtained by transforming E. coli MM294(DE3) with the resulting expression plasmid was cultivated in NZCYM medium [M. Maniatis et al., Molecular Cloning, page 440, Cold Spring Harbor Laboratory (1982)], and gene expression was induced with 1 mM isopropyl thiogalactoside (IPTG). The gene product formed an inclusion body as a fused protein (37 kDal) of T7 phage gene 10 (260 amino acid residues) and xBMP4 (70 amino acid residues) to be accumulated in E. coli. E. coli was disrupted by ultrasonication, and the cell debris was removed with gauze, followed by collection of the inclusion bodies by centrifugation. The fused protein was suspended with Freund's adjuvant, and then, rabbits were subcutaneously immunized therewith three times at intervals of two weeks. At one week after three inoculations, the blood was collected to prepare a serum. In this serum, an anti-xBMP4 antibody was detected in high titers.

### Example 2

### Preparation of Expression Plasmids (Baculovirus Transfer Plasmids) of xBMP4 and xBMP7

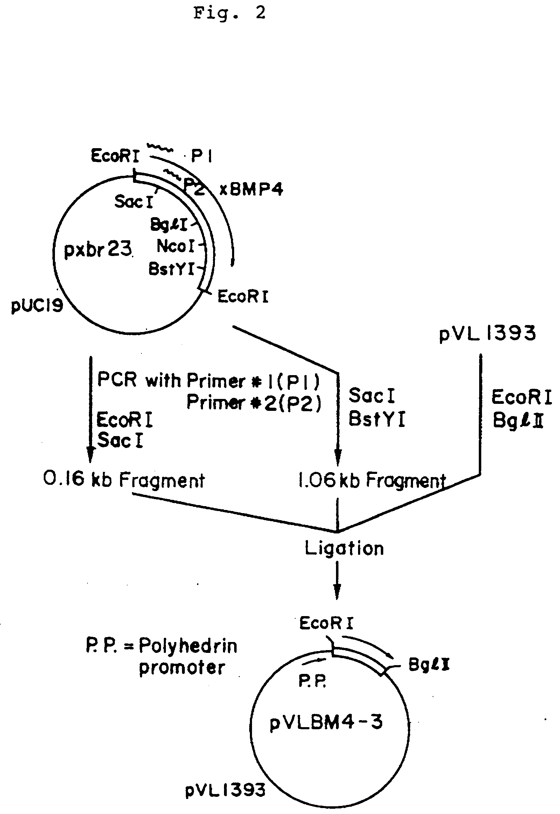

Using cDNA of xBMP4 (pXbr23, Japanese Patent Unexamined Publication No. 4-154799) as a template, PCR was conducted using a sense primer #1 (GGAATTCACCATGGTTCCTGGTAACCGA; SEQ ID NO: 2), an anti-sense primer #2 (CTCGAGAACGCCCTAAAGCTCCAC; SEQ ID NO: 3) and a PCR kit (GeneAmp, Perkin Elmer Cetus: Norwalk, CT, U.S.A.). The amplified DNA fragment was cleaved with EcoRI and SacI to isolate a 0.16-kb fragment. Further, the cDNA was cleaved with SacI and BstYI to isolate a 1.06-kb fragment. These fragments were ligated with Baculovirus transfer vector pVL1393 (Invitrogen Corporation, San Diego, CA, U.S.A.) in which the vector was opened with EcoRI and BglII, using T4 DNA ligase, and the resulting plasmid (pVLBM4-3; IFO 15486, FERM BP-4312) was applied to a preparation of a recombinant virus (Fig. 2).

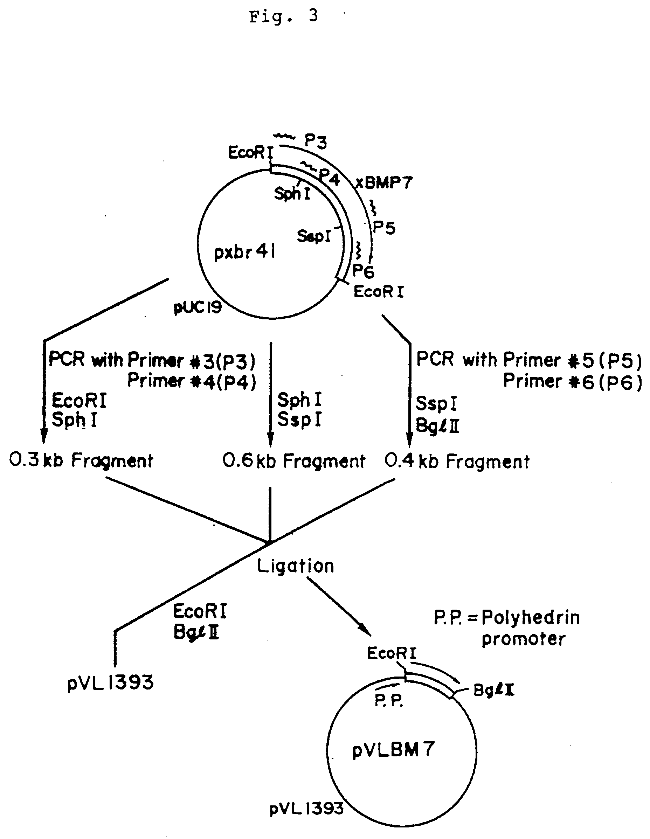

Using a cDNA of xBMP7 (pXbr41, Japanese Patent Unexamined Publication No. 4-154799) as a template, PCR was conducted using a sense primer #3 (GGAATTCAAAATGAATGCTTTGACAGTAAAG; SEQ ID NO: 4), an anti-sense primer #4 (GGAAACATCTTCAGCATGCATCTC; SEQ ID NO: 5) and a PCR kit. The amplified DNA fragment was cleaved with EcoRI and SphI to isolate a 0.3-kb fragment. Further, the cDNA was cleaved with SphI and SspI to isolate a 0.6-kb fragment. Furthermore, using a cDNA of xBMP7 as a template, PCR was conducted using a sense primer #5 (GATAATTTACCTCCAGCAAATATT; SEQ ID NO: 6), an anti-sense primer #6 (GAAGATCTCAATGGCAACCACAGGCTTGAAC; SEQ ID NO: 7) and a PCR kit. The amplified DNA fragment was cleaved with SspI and BglII to isolate a 0.4-kb fragment. These fragments were ligated with Baculovirus transfer vector pVL1393 (Invitrogen Corporation, San Diego, CA, U.S.A.) in which the vector was opened with EcoRI and BglII, using T4 DNA ligase, and the resulting plasmid (pVLBM7; IFO 15487, FERM BP-4314) was applied to a preparation of a recombinant virus (Fig. 3).

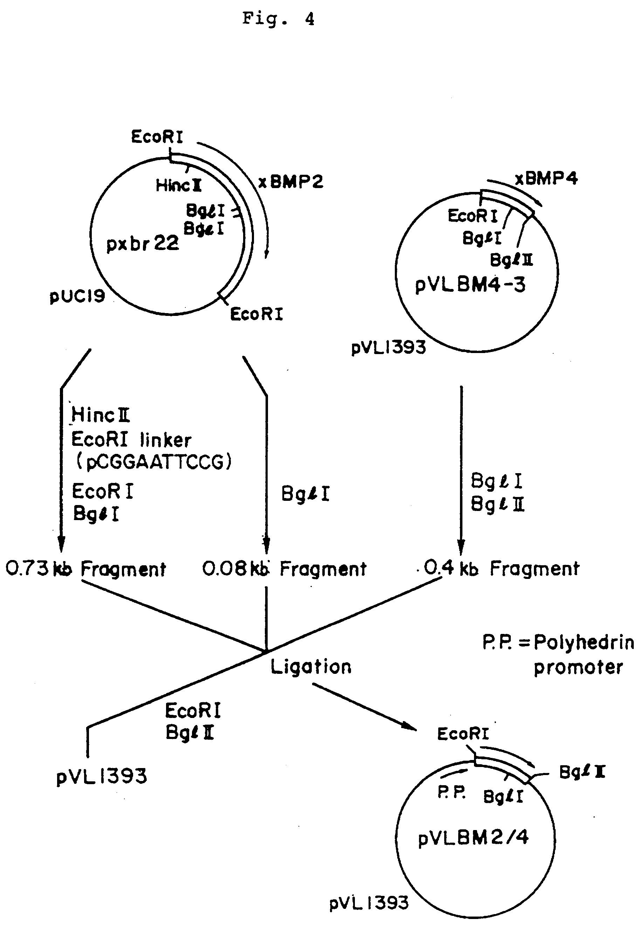

A cDNA of xBMP2 (pXbr22, Japanese Patent Unexamined Publication No. 4-154799) was cleaved with HincII, and an EcoRI linker (pCGGAATTCCG) was added thereto, followed by cleavage with EcoRI and BglII to obtain 0.73-kb and 0.08-kb fragments. pVLB4-3 was cleaved with BglI and BglII to obtain a 0.4 kb fragment. These three fragments were ligated with pVL1393 in which the vector was opened with EcoRI and BglII, using T4 DNA ligase, and the resulting plasmid (pVLBM2/4; the pro-region of xBMP2 was used; IFO 15488, FERM BP-4313) was applied to a preparation of a recombinant virus (Fig. 4).

### Example 3

### Preparation of Recombinant Viruses

Each of the Baculovirus transfer plasmids (pVLBM4-3 and pVLBM7) obtained in Example 2 was introduced into an Sf9 insect cell together with a wild type Baculovirus genome DNA by calcium phosphate coprecipitation. After 4 days, the culture supernatant was recovered, and diluted 10⁷ times. Then, 10 µl of the diluted supernatant was added to each well of a 96-well plate, and 10⁴ Sf9 insect cells were added thereto. After 7 days, a cell infected with a recombinant virus was searched under a microscope, and the culture supernatant was recovered from the detected wells. The supernatant was diluted in the same manner as above, and Sf9 insect cells were infected therewith to purify the recombinant virus. Then, 150 ml of Sf9 insect cells were infected with each of the thus purified recombinant viruses (BVBM4 and BVBM7) for expressing xBMP4 and xBMP7, respectively. After cultivation for 4 days, the culture supernatants were stored as virus stock solutions at 4°C until they were to be used.

### Example 4

### Expression of xBMP4 by recombinant viruses

In spinner flasks into which Grace's insect medium (Invitrogen Corporation) had been dispensed, Sf9 insect cells proliferated to 2X10⁶ cells/ml were infected with recombinant viruses BVBM4 and BVBM7 obtained in Example 3, individually or concurrently (multiplicity of infection (moi) = 10), followed by dispensing into 15-cm culture dishes. After 24 hours, the culture solution was changed with a TNM-FH culture solution [W. F. Hink, Nature, 226, 466 (1970)], and cultivation was further continued for 36 hours, followed by recovery of a culture supernatant. The resulting supernatant was filtered through a 0.2-µm membrane filter (Kurabo Industries, Neyagawa, Osaka), and subjected to purification.

### Example 5

### Purification of xBMP4

Urea was added to 4 liters of the culture supernatant of the Sf9 insect cells infected with BVBM4, which was prepared in Example 4, to give a final concentration of 4 M, and the resulting solution was loaded onto a 200-ml SP-Sepharose column (Pharmacia) equilibrated with 4 M urea/25 mM Tris-HCl (pH 8.0). After washed with the above-mentioned buffer, the column was eluted with a linear gradient of 0 to 0.6 M NaCl, and xBMP4 fractions (about 200 ml) eluted in the vicinity of 0.4 M NaCl were collected. The fractions were dialyzed against 4 M urea/25 mM Tris-HCl (pH 7.0), and then, loaded onto a 10-ml heparin-Sepharose column equilibrated with the same buffer. After the column was washed with the same buffer containing 0.2 M NaCl, xBMP4 (about 60 ml) was eluted with the same buffer containing 1 M NaCl. The eluate was subjected to ultrafiltration using a Centricut Fraction 10,000 filter (Kurabo Industries), and concentrated to 7 ml. The concentrated solution was applied to reversed phase HPLC using a µ-Bondasphere C4-300A (Waters) column, and the column was eluted with a linear gradient of 30 to 40% acetonitrile in the presence of 0.1% trifluoroacetic acid (TFA) to recover a xBMP4 fraction having a somewhat wide single peak. This fraction was freeze-dried by Servant (Instrument Inc., USA), and thereafter dissolved in 0.2 N acetic acid to obtain 2 ml of a 180 µg/ml solution of xBMP4.

The resulting xBMP4 solution was subjected to SDS-PAGE under non-reducing and reducing conditions, followed by confirmation according to CBB (coomassie brilliant blue) staining and Western blot analysis using rabbit anti-BMP4 antisera. As a result, xBMP4 was detected as a band corresponding to a molecular weight of about 34 to 35K under the non-reducing conditions, and as two bands corresponding to molecular weights of about 19 K and about 17.5 K, respectively, under the reducing conditions by CBB staining. All of these bands were confirmed by the anti-xBMP4 antibody. It was confirmed from the results described above that xBMP4 formed a homodimer by an S-S bond.

### Example 6

### Purification of xBMP7

Urea was added to 4 liters of the culture supernatant of the Sf9 insect cells infected with BVBM7, which was prepared in Example 4, to give a final concentration of 4 M, and the resulting solution was loaded onto a 200-ml SP-Sepharose column (Pharmacia) equilibrated with 4 M urea/25 mM Tris-HCl (pH 8.0). After washed with the above-mentioned buffer, the column was eluted with a linear gradient of 0 to 0.6 M NaCl, and xBMP7 fractions (about 200 ml) eluted in the vicinity of 0.4 M NaCl were collected. The fractions were dialyzed against 4 M urea/25 mM Tris-HCl (pH 7.0), and then, loaded onto a 10-ml heparin-Sepharose column equilibrated with the same buffer. After the column was washed with the same buffer containing 0.2 M NaCl, xBMP7 (about 60 ml) was eluted with the same buffer containing 1 M NaCl. The eluate was subjected to ultrafiltration using a Centricut Fraction 10,000 filter (Kurabo Industries), and concentrated to 7 ml. The concentrated solution was applied to reversed phase HPLC using a µ-Bondasphere C4-300A (Waters) column, and the column was eluted with a linear gradient of 30 to 40% acetonitrile in the presence of 0.1% TFA to recover a xBMP7 fraction having a somewhat wide single peak. This fraction was dried by the use of Servant, and thereafter dissolved in 0.2 N acetic acid to obtain 2 ml of a 100 µg/ml solution of xBMP-7.

The resulting xBMP7 solution was subjected to SDS-PAGE under non-reducing and reducing conditions, followed by CBB staining. As a result, xBMP7 behaved as a band corresponding to a molecular weight of about 37 to 38 K under the non-reducing conditions, and as two bands corresponding to molecular weights of about 23 to 24 K and about 21 K, respectively, under the reducing conditions by CBB staining. These bands of xBMP7 were all weakly recongnized by the anti-xBMP4 antibody. It was confirmed from the results described above that xBMP7 formed a homodimer by an S-S bond.

### Example 7

### Purification of Heterodimer composed of xBMP4 and xBMP7

Urea was added to 1 liter of the culture supernatant of the Sf9 insect cells concurrently infected with BVBM4 and BVBM7, which were prepared in Example 4, to give a final concentration of 4 M, and the resulting solution was loaded onto a 100-ml SP-Sepharose column (Pharmacia) equilibrated with 4 M urea/25 mM Tris-HCl (pH 8.0). After washing with the above-mentioned buffer, the column was eluted with a linear gradient of 0 to 0.6 M NaCl, and fractions (about 90 ml) of heterodimers composed of xBMP4 and xBMP7 (xBMP4/7 heterodimers) eluted in the vicinity of 0.2 M NaCl were collected. The fractions were subjected to ultrafiltration using a Centricut Fraction 10,000 filter (Kurabo Industries), and concentrated to about 7 ml. The concentrated solution was applied to reversed phase HPLC using a µ-Bondasphere C4-300A (Waters) column, and the column was eluted with a linear gradient of 30 to 42% acetonitrile in the presence of 0.1% TFA. The xBMP4/7 heterodimer fraction eluted as a somewhat wide single peak was dried by the use of Servant, and thereafter dissolved in 0.2 N acetic acid to obtain 2.5 ml of a 160 µg/ml solution thereof.

The resulting xBMP4/7 heterodimer was subjected to SDS-PAGE under non-reducing and reducing conditions, followed by detection of the heterodimer according to CBB staining and Western blot analysis using rabbit anti-BMP4 antisera. As a result, for the xBMP4/7 heterodimer, a band corresponding to a molecular weight of about 36 K was detected under the non-reducing conditions, and four bands corresponding to molecular weights of about 23 to 24 K, about 21 K, about 19 K and about 17.5 K, respectively, under the reducing conditions by CBB staining. The bands corresponding to molecular weights of about 19 K and about 17.5 K, respectively, which were detected under the reducing conditions were observed also when a homodimer of xBMP4 was reduced, and clearly recognized by the anti-xBMP4 antibody. From these facts, these bands were considered to correspond to xBMP4. The remaining bands corresponding to molecular weights of about 23 to 24 K and about 21 K, respectively, which were detected under the reducing conditions were not observed when a homodimer of xBMP4 was reduced, and further weakly recognized by the anti-xBMP4 antibody. From these facts, these bands were considered to correspond to xBMP7. The band corresponding to a molecular weight of about 36 K detected under the non-reducing conditions was larger than that of the xBMP4 homodimer (about 34 to 35 K), and recognized by the anti-xBMP4 antibody, from which this band was considered to correspond to the xBMP4/7 heterodimer.

### Experimental Example 1

### Assay for Alkaline Phosphatase Induced by xBMP4, xBMP7 and xBMP4/7 in Mouse Osteoblastic Cell

2X10⁴ mouse-derived MC3T3-E1 osteoblast cells were cultivated on a 24-well plate for 4 days. At the time when the cells reached a subconfluent state, each of the solutions of the xBMP4 homodimers, the xBMP7 homodimers and the xBMP4/7 heterodimers obtained in Examples 5, 6 and 7, respectively, which were diluted with alpha MEM medium supplemented with 0.3% FCS to provide various concentrations, was added thereto, and cultivation was further continued for 2 days. The culture medium was removed, and the cells which adhered to the plate were washed with phosphate buffered saline (PBS). Then, 0.15 ml of a 0.2% NP-40 solution was added to the plate, followed by freeze-thaw twice. The disrupted-cell solution obtained by this procedure was centrifuged to remove insoluble materials. 0.04 ml of the resulting supernatant was mixed with 0.01 ml of a substrate solution (33.5 mmol/L disodium p-nitrophenylphosphate, 0.5 M carbonate buffer; pH 9.8), and the temperature of the mixture was maintained 37°C for 30 minutes. Then, 0.3 ml of 0.05 N NaOH was added thereto to terminate the reaction, and the absorbance at a wavelength of 405 nm was measured. The results proved that the xBMP4/7 heterodimer had a specific activity several times higher than xBMP4 and xBMP7 homodimer, as is shown in Table 2.

**TABLE 2**

| BMP | Alkaline phosphatase activity (A₄₀₅) | | | |
|---|---|---|---|---|
| | BMP concentration (ng/ml) | | | |
| | 0.3 | 1 | 3 | 10 |
| xBMP4 | 0.036 | 0.060 | 0.092 | 0.193 |
| xBMP4/7 | 0.083 | 0.192 | 0.377 | 0.692 |
| xBMP7 | 0.047 | 0.037 | 0.040 | 0.039 |

### Experimental Example 2

### Assay for Ectopic Bone-inducing Activity

Purified samples of the xBMP4 homodimer and the xBMP4/7 heterodimer prepared in Examples 5 and 7, respectively, were subcutaneously transplanted to the breast of rats, and ectopic bone-inducing activity was evaluated, in accordance with Experimental Example described in Japanese Patent Unexamined Publication No. 5-85939. Namely, these samples were dried again, and then dissolved into 0.1% (v/v) TFA to give a concentration of 1 mg/ml. Using low immunogenic liquid collagen (Cellmatrix LA, Nitta Gelatin) as a carrier, xBMP was added to 5 ml of a collagen solution to bring the dose to 0 ug, 10 ug or 30 ug, and the mixture was stirred at 4°C for 1 hour. The resulting solution was neutralized with 0.1 N NaOH, and lyophilized, followed by compression to form a pellet. The pellet was subcutaneously transplanted to each of the breasts of two 4-week-old male rats, for every xBMP and dose. After 3 weeks, implants were harvested, and ectopic bone formation was confirmed by soft X-ray photographic images of the implants and observation of tissue sections. Further, the calcium and phosphorus contents of calcified implants were assayed, thereby evaluating the degree of ectopic bone formation. The result proved that the xBMP4/7 heterodimer was clearly higher in ectopic bone-inducing activity than the xBMP4 homodimer, as is shown in Table 3.

**TABLE 3**

| xBMP amount (µg) | xBMP4 homodimer | | xBMP4/7 heterodimer | |
|---|---|---|---|---|
| | Ca (mg) | P (mg) | Ca (mg) | P (mg) |
| 0 | 0.059 | 0.062 | - | - |
| | 0.046 | 0.085 | - | - |
| 10 | 0.263 | 0.203 | 1.365 | 0.896 |
| | 0.330 | 0.284 | 0.925 | 0.564 |
| 30 | 2.305 | 1.297 | 4.084 | 2.117 |
| | 1.240 | 0.803 | 4.412 | 2.331 |

### Example 8

### Preparation of Expression Plasmid (Baculovirus Transfer Plasmid) for Drosophiladecapentapregic (DPP) Gene

A drosophiladecapentapregic (DPP) gene was cloned by the RT-PCR method using poly(A)⁺ RNA as a template, based on the cDNA nucleotide sequence previously reported[R. W. Padgett et al., Nature, 325, 81 (1987)]. Namely, RNA fractions were recovered from 1 g of embryos of 3 to 12 hours according to the method of S. J. Poole et al. [Cell, 40, 37 (1985), and poly(A)⁺ RNA was purified by an oligo(dT) cellulose column (Pharmacia LKB, Uppsala, Sweden). Using this RNA as a template, PCR was conducted by the use of a RT-PCR kit (Pharmacia LKB), a sense primer #7 (GGAATTCACCATGCGC-GCATGGCTTCTACT; SEQ ID NO: 8) and an anti-sense primer #8 (GAAGATCTATCGACAGCCACAGCCCACCAC; SEQ ID NO: 9). An amplified DNA fragment was cleaved with EcoRI and BglII, and the resulting 1.8-kb fragment was isolated. This fragment was ligated with Baculovirus transfer vector pVL1393, in which the vector was opened with EcoRI and BglII, by the use of T4 DNA ligase, and the resulting plasmid (pVLDPP) was applied to a preparation of a recombinant virus.

### Example 9

### Preparation of Recombinant Virus

The Baculovirus transfer plasmid (pVLDPP) obtained in Example 8 was introduced into an Sf9 insect cell together with a wild type Baculovirus genome DNA by calcium phosphate coprecipitation. After 4 days, the culture supernatant was recovered, and diluted 10⁷ times. Then, 10 µl of the diluted supernatant was added to each well of a 96-well plate, and 10⁴ Sf9 insect cells were added thereto. After 7 days, cell infected with a recombinant virus were detected using a microscope, and the culture supernatant was recovered from the detected wells. The supernatant was diluted in the same manner as above, and Sf9 insect cells were infected therewith to purify the recombinant virus. Then, 150 ml of Sf9 insect cells were infected with the thus purified recombinant virus (BVDPP) for expressing DPP. After cultivation for 4 days, the culture supernatant was stored as a virus stock solution at 4°C until it is to be used.

### Example 10

### Expression of xBMP and DPP by Recombinant Viruses

In spinner flasks into which Grace's insect medium (Invitrogen Corporation) had been dispensed, Sf9 insect cells proliferated to 2X10⁶ cells/ml were infected with recombinant viruses BVBM4 and BVBM7 obtained in Example 3, and recombinant virus BVDPP obtained in Example 9, individually, or concurrently with BVBM4 and BVBM7, or concurrently with BVDPP and BVBM7 (moi = 10), followed by dispensing into 15-cm culture dishes. After 24 hours, the culture solution was changed for a TNM-FH culture solution [W. F. Hink, Nature, 226, 466 (1970)], and cultivation was further continued for 36 hours, followed by recovery of a culture supernatant. The resulting supernatant was filtered through a 0.2-µm membrane filter (Kurabo Industries, Neyagawa, Osaka).

### Experimental Example 3

### Assay for Alkaline Phosphatase Induced by DPP Homodimer and DPP/xBMP7 Heterodimer in Mouse Osteoblastic Cells

The assay for the activity of the DPP homodimer and the DPP/xBMP7 heterodimer was conducted according to the method described in Experimental Example 1 mentioned above. The culture supernatant containing the expressed product obtained in Example 10 was added to mouse MC3T3-E1 osteoblastic cells in an amount of 15 µl, and the activity after 24 hours was assayed. The result proved that the DPP/xBMP7 heterodimer had an activity similar to that of the xBMP4/7 heterodimer, as is shown in Table 4.

**TABLE 4**

| Culture supernatant | Alkaline phosphatase activity (A₄₀₅) |
|---|---|
| Control | 0.030 |
| xBMP4 homodimer | 0.245 |
| xBMP7 homodimer | 0.076 |
| DPP homodimer | 0.270 |
| xBMP4/7 heterodimer | 0.448 |
| DPP/xBMP7 heterodimer | 0.450 |

### Experimental Example 4

### Assay for Ectopic Bone-inducing Activity

Purified samples of the xBMP4 homodimers, xBMP7 homodimers and the xBMP4/7 heterodimers prepared in Examples 5, 6 and 7, respectively, were subcutaneously transplanted to the breasts of rats, and ectopic bone-inducing activity was evaluated, in accordance with Experimental Example described in Japanese Patent Unexamined Publication No. 5-85939 (Notoya et al.). Namely, these examples were dried again, and then dissolved in 0.1% (v/v) trifluoroacetic acid to give a concentration of 1 mg/ml. Using low immunogenic liquid collagen (Cellmatrix LA, Nitta Gelatin) as a substrate, each xBMP sample was added to 5 ml (13.5 mg) of a collagen solution to bring the dose to 0 µg, 0.1 µg, 0.3 µg, 1 µg, 3 µg, 10 µg or 30 µg, and the mixture was stirred at 4°C for 1 hour. The resulting solution was neutralized with 0.1 N NaOH, and lyophilized, followed by compression to form 6 pellets. The pellets were subcutaneously transplanted to the breasts of 5-week-old male rats. After 3 weeks, implants were harvested, and ectopic bone formation was confirmed by soft X-ray photographic images of the implants and observation of tissue sections. Further, the calcium content of calcified implants was assayed, thereby evaluating the degree of ectopic bone formation. The result proved that the xBMP4/7 heterodimer was clearly higher in ectopic bone-inducing activity than the xBMP4 homodimer, the xBMP7 homodimer, and the mixture of the xBMP4 homodimer and the xBMP7 homodimer, as is shown in Table 5.

**TABLE 5**

| xBMP amount (µg) | xBMP4 homodimer | xBMP7 homodimer | Mixture of xBMP4 and 7 homodimers | xBMP4/7 heterodimer |
|---|---|---|---|---|
| | Ca content mg (SE) | Ca content mg (SE) | Ca content mg (SE) | Ca content mg (SE) |
| 0 | 0.070(0.015) | - | - | - |
| 0.1 | 0.099(0.019) | 0.114(0.015) | - | 0.098(0.005) |
| 0.3 | 0.094(0.016) | 0.090(0.011) | - | 0.086(0.018) |
| 1.0 | 0.100(0.007) | 0.082(0.015) | - | 0.552(0.130) |
| 3.0 | 0.071(0.008) | 0.053(0.013) | 0.078(0.017) | 1.024(0.112) |
| 10.0 | 0.358(0.145) | 0.486(0.122) | - | 1.704(0.162) |
| 30.0 | 0.730(0.157) | 0.924(0.119) | - | 2.231(0.272) |

### Experimental Example 5

### Assay for Alkaline Phosphatase Induced by xBMP4 Homodimer, xBMP7 Homodimer, xBMP4/7 Heterodimer and Mixture of xBMP4 and 7 Homodimers in Rat Femur Bone Marrow

### Interstitial Cells

The bone marrow obtained from the femurs of 5-week-old male SD rats was collected on a 10-ml culture dish containing complete medium (α-MEM, 20% FCS, 10 mM Na-β-glycerophosphoric acid, 2 mM glutamine, 50 µg/ml ascorbic acid). The culture solution was washed with the above-mentioned complete medium every day to remove cells not adhered. After 1 week, cells separated by trypsin treatment were poured into each well of a 24-well plate so as to provide 1X10⁵ cells/well. After 24 hours, the medium was changed with α-MEM medium containing 0.5% FCS and each of the various xBMP samples. After 2 days, the cells were washed with physiological saline, followed by addition of 0.15 ml of a 0.2% NP-40 solution to the plate. Then, lyophilization was repeated twice to disrupt the cells. The disrupted-cell solution was centrifuged, and 0.04 ml of the resulting supernatant was mixed with 0.01 ml of a substrate solution (33.5 mmol/L disodium p-nitrophenyl-phosphate, 0.5 M carbonate buffer; pH 9.8), and the temperature of the mixture was maintained 37°C for 30 minutes. Then, 0.3 ml of 0.05 N NaOH was added thereto to terminate the reaction, and the absorbance at a wavelength of 405 nm was measured. Based on the absorbance obtained by the standard solution of p-nitrophenol, the alkaline phosphatase activity was calculated. Results obtained when the various xBMP samples were added in an amount of 100 ng/ml are shown in Table 6. The results shown in Table 6 proved that the xBMP4/7 heterodimer had a specific activity higher than each of the xBMP4 and 7 homodimers even in bone marrow interstitial cells.

**TABLE 6**

| Supernatant | Alkaline phosphatase activity (p-nitrophenol nmol/min/well) |
|---|---|
| Control | 309 |
| xBMP4 homodimer | 565 |
| xBMP7 homodimer | 1200 |
| xBMP4 homodimer + xBMP7 homodimer | 1289 |
| xBMP4/7 heterodimer | 1980 |

The heterodimers of the TGF-β super families of the present invention promote ectopic bone formation because of their high bone-inducing promoting activity, and can therefore be used as therapeutic agents for bone diseases, for example, bone-inducing agents when the bones are repaired or transplanted.

## Claims

1. An expression vector which is autonomically replicable in an insect cell and comprises one or more DNA(s) coding for a factor(s) belonging to a TGF-β superfamily.

2. An expression vector which is autonomically replicable in an insect cell and comprises DNAs coding for two factors belonging to a TGF-β superfamily.

3. The expression vector according to claim 1 or 2, wherein the TGF-β superfamily includes a bone morphogenetic protein family, a TGF-β family and an inhibin family.

4. The expression vector according to claim 3, wherein the bone morphogenetic protein family includes bone morphogenetic factor 2, 3, 4, 5, 6, 7, 8 and 9, XenopusVg1, drosophiladecapentapregic and drosophila 60A.

5. The expression vector according to claim 3, wherein the TGF-β family includes TGF-β₁, β₂, β₃, β₄ and β₅.

6. The expression vector according to claim 3, wherein the inhibin family includes inhibin α, β_{A} and β_{B}.

7. The expression vector according to claim 1 or 2, which is a Baculovirus vector.

8. The expression vector according to claim 1 or 2, wherein the insect cell is a Spodoptera litura-derived cell or a spanworm-derived cell.

9. An insect cell transformed with the expression vector according to claim 1.

10. An insect cell transformed with the expression vector according to claim 2.

11. An insect cell transformed with an expression vector which is autonomically replicable in an insect cell and comprises a DNA coding for a factor belonging to a TGF-β superfamily and an expression vector which is autonomically replicable in an insect cell and comprises a DNA coding for a factor belonging to a TGF-β superfamily other than the above said factor.

12. A method for preparing a heterodimer composed of two factors belonging to a TGF-β superfamily comprising the steps of cultivating the insect cell of claim 9, 10 or 11 in a medium under conditions sufficient to express the heterodimer, accumulating the heterodimer in a culture product, and collecting the resulting accumulated heterodimer.

13. A heterodimer composed of two factors belonging to a TGF-β superfamily in which the first factor is drosophiladecapentapregic and the second factor is a factor belonging to a TGF-β superfamily other than the first factor.

14. The heterodimer according to claim 13, wherein the second factor is selected from the group consisting of bone morphogenetic protein 2, 3, 4, 5, 6, 7, 8 and 9, XenopusVg1 and drosophila 60A.

15. The heterodimer according to claim 14, wherein the second factor is bone morphogenetic protein 4, 6 or 7.

16. The heterodimer according to claim 15, wherein the second factor is bone morphogenetic protein 7.

17. A heterodimer composed of two factors belonging to a bone morphogenetic protein family which is derived from Xenopus laevis.

18. The heterodimer according to claim 17, wherein the bone morphogenetic protein family includes bone morphogenetic protein 2, 3, 4, 5, 6, 7, 8 and 9, XenopusVg1, drosophiladecapentapregic and drosophila 60A.

19. The heterodimer according to claim 18, wherein the bone morphogenetic protein family is bone morphogenetic protein 4, 6 or 7.

20. The heterodimer according to claim 19, wherein the bone morphogenetic protein family is bone morphogenetic protein 4 and 6.

21. The heterodimer according to claim 19, wherein the bone morphogenetic protein family is bone morphogenetic protein 4 and 7.

22. A composition for osteogenesis which comrises a pharmaceutically acceptable carrier containing an effective amount of a heterodimer composed of two factors belonging to a TGF-β superfamily in which the first factor is drosophiladecapentapregic and the second factor is a factor belonging to a TGF-β superfamily other than the first factor.

23. The composition according to claim 22, wherein the second factor is selected from the group consisting of bone morphogenetic protein 2, 3, 4, 5, 6, 7, 8 and 9, XenopusVg1 and drosophila 60A.

24. The composition according to claim 23, wherein the second factor is bone morphogenetic protein 4, 6 or 7.

25. The composition according to claim 24, wherein the second factor is bone morphogenetic protein 7.

26. A composition for osteogenesis which comprises a pharmaceutically acceptable carrier containing an effective amount of a heterodimer composed of two factors belonging to a bone morphogenetic protein family which is derived from Xenopus laevis.

27. The composition according to claim 26, wherein the bone morphogenetic protein family includes bone morphogenetic protein 2, 3, 4, 5, 6, 7, 8 and 9, XenopusVg1, drosophiladecapentapregic and drosophila 60A.

28. The composition according to claim 27, wherein the bone morphogenetic protein family is bone morphogenetic protein 4, 6 or 7.

29. The composition according to claim 28, wherein the bone morphogenetic protein family is consisting ofbone morphogenetic protein 4 and 6.

30. The composition according to claim 28, wherein the bone morphogenetic protein family is consisting ofbone morphogenetic protein 4 and 7.

31. A composition for therapy of osteopathy which comprises a pharmaceutically acceptable carrier containing an effective amount of a heterodimer composed of two factors belonging to a TGF-β superfamily in which the first factor is drosophiladecapentapregic and the second factor is a factor belonging to a TGF-β superfamily other than the first factor.

32. A composition for therapy of osteopathy which comprises a pharmaceutically acceptable carrier containing an effective amount of a heterodimer composed of two factors belonging to a bone morphogenetic protein family which is derived from Xenopus laevis.

33. An assay for determining the effect of a test compound on alkaline phosphatase induction in an osteoblast comprising:
(a) contacting an osteoblast with a heterodimer according to claim 13 and measuring alkaline phosphatase induction;
(b) contacting the osteoblast of step (a) with the test compound; and
(c) measuring the change in alkaline phosphatase induction.


SEQUENCE LISTING

(2) INFORMATION FOR SEQ ID NO:1:

(i) SEQUENCE CHARACTERISTICS:
(A) LENGTH: 10 base pairs
(B) TYPE: nucleic acid
(C) STRANDEDNESS: single
(D) TOPOLOGY: linear

(ii) MOLECULE TYPE: Other nucleic acid; synthetic DNA

(iv) ANTI-SENSE: NO

(xi) SEQUENCE DESCRIPTION: SEQ ID NO:1:

CGGGATCCCG 10

(2) INFORMATION FOR SEQ ID NO:2:

(i) SEQUENCE CHARACTERISTICS:
(A) LENGTH: 28 base pairs
(B) TYPE: nucleic acid
(C) STRANDEDNESS: single
(D) TOPOLOGY: linear

(ii) MOLECULE TYPE: Other nucleic acid; synthetic DNA

(iv) ANTI-SENSE: NO

(xi) SEQUENCE DESCRIPTION: SEQ ID NO:2:

GGAATTCACC ATGGTTCCTG GTAACCGA 28

(2) INFORMATION FOR SEQ ID NO:3:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: Other nucleic acid; synthetic DNA

(iv) ANTI-SENSE: YES

(xi) SEQUENCE DESCRIPTION: SEQ ID NO:3:

CTCGAGAACG CCCTAAAGCT CCAC 24

(2) INFORMATION FOR SEQ ID NO:4:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: Other nucleic acid; synthetic DNA

(iv) ANTI-SENSE: NO

(xi) SEQUENCE DESCRIPTION: SEQ ID NO:4:

GGAATTCAAA ATGAATGCTT TGACAGTAAA G 31

(2) INFORMATION FOR SEQ ID NO:5:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: Other nucleic acid; synthetic DNA

(iv) ANTI-SENSE: YES

(xi) SEQUENCE DESCRIPTION: SEQ ID NO:5:

GGAAACATCT TCAGCATGCA TCTC 24

(2) INFORMATION FOR SEQ ID NO:6:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: Other nucleic acid; synthetic DNA

(iv) ANTI-SENSE: NO

(xi) SEQUENCE DESCRIPTION: SEQ ID NO:6:

GATAATTTAC CTCCAGCAAA TATT 24

(2) INFORMATION FOR SEQ ID NO:7:

(i) SEQUENCE CHARACTERISTICS:
(A) LENGTH: 31 base pairs
(B) TYPE: nucleic acid
(C) STRANDEDNESS: single
(D) TOPOLOGY: linear

(ii) MOLECULE TYPE: Other nucleic acid; synthetic DNA

(iv) ANTI-SENSE: NO

(xi) SEQUENCE DESCRIPTION: SEQ ID NO:7:

GAAGATCTCA ATGGCAACCA CAGGCTTGAA C
31

(2) INFORMATION FOR SEQ ID NO:8:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: Other nucleic acid; synthetic DNA

(iv) ANTI-SENSE: NO

(xi) SEQUENCE DESCRIPTION: SEQ ID NO:8:

GGAATTCACC ATGCGCGCAT GGCTTCTACT 30

(2) INFORMATION FOR SEQ ID NO:9:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: Other nucleic acid; synthetic DNA

(iv) ANTI-SENSE: NO

(xi) SEQUENCE DESCRIPTION: SEQ ID NO:9:

GAAGATCTAT CGACAGCCAC AGCCCACCAC 30